# EUROPÄISCHE PATENTSCHRIFT

(11) **EP 1 175 382 B1**
(45) Veröffentlichungstag und Bekanntmachung des Hinweises auf die Patenterteilung: **09.02.2005**
(21) Anmeldenummer: 00926978.8
(22) Anmeldetag: 18.04.2000
(51) Int. Cl.: C07C 29/50, C07C 31/20, C07C 31/42, C07C 33/26, C07C 35/14, C07C 35/21, C07C 41/26, C07C 43/253, C07C 319/20, C07C 323/12, C07D 319/06, C07F 7/08

(54) **VERFAHREN ZUR DIHYDROXYLIERUNG VON OLEFINEN MITTELS ÜBERGANGSMETALL-KATALYSATOREN**
METHOD FOR THE DIHYDROXYLATION OF OLEFINS USING TRANSITION-METAL CATALYSTS
PROCEDE DE DIHYDROXYLATION D'OLEFINES A L'AIDE DE CATALYSEURS A BASE DE METAUX DE TRANSITION

(30) Priorität: 25.04.1999 DE 19920038
(43) Veröffentlichungstag der Anmeldung: 30.01.2002
(73) Patentinhaber: Bayer Chemicals AG, 51368 Leverkusen (DE)
(72) Erfinder: BELLER, Matthias, D-18119 Rostock (DE); DÖBLER, Christian, D-18107 Lichtenhagen-Dorf (DE); MEHLTRETTER, Gerald, D-18057 Rostock (DE); SUNDERMEIER, Uta, D-18055 Rostock (DE)
(86) Internationale Anmeldenummer: PCT/EP2000/003493
(87) Internationale Veröffentlichungsnummer: WO 2000/064848

(56) Entgegenhaltungen:
- EP-A- 0 077 201
- US-A- 3 317 592
- T. BAROW, ET AL.: "Arylation of guanosine with para-substituted styrene oxides" CHEMICAL RESEARCH IN TOXICOLOGY, Bd. 11, Nr. 1, Januar 1998 (1998-01), Seiten 44-53, XP000929704 American Chemical Society, Washington, DC, US11

## Beschreibung

Die vorliegende Erfindung betrifft ein Verfahren zur Herstellung von 1,2-Diolen aus Olefinen mit Katalysatoren auf Basis von Übergangsmetallverbindungen.

1,2-Diole, insbesondere cis-1,2-Diole, haben technische Bedeutung als Feinchemikalien, Lösemittel, Ausgangsprodukte für Polyester und andere Polymere, sowie als Vorprodukte für Agrochemikalien. Von außerordentlicher Bedeutung sind besonders Propylenglykol und Ethylenglykol als Bulkchemikatien. Zahlreiche 1,2-Diole sind auch von kommerziellem Interesse für die Darstellung von Arzneimitteln, Kosmetika, Reinigungsmitteln und finden Anwendung in der Textil - und Plasteindustrie. Die Carbonsäureester zeigen in vielen Fällen konstante Viskosität über weite Temperaturbereiche, verbunden mit einem hohen Siedepunkt. Sie sind gute synthetische Schmierstoffe und Weichmacher.

Eine häufig angewandte Methode zur Synthese von 1,2-Diolen im universitären Bereich sind sogenannte "Dihydroxylierungsreaktionen" wie die Sharpless-Dihydroxylierungsreaktion, bei der Olefine in Gegenwart von Osmiumtetroxid und einem Oxidationsmittel umgesetzt werden. Übersichtsartikel, die diese Methodik beschreiben, findet man beispielsweise in *"Asymmetric Dihydroxylation Reactions"* M. Beller, K. B. Sharpless, in B. Cornils, W.A. Herrmann (Eds.), VCH, 1996, Weinheim und H.C. Kolb, M.S. Van Nieuwenhze, K.B. Sharpless, *Chem. Rev.* 1994, *94*, 2483.

Olefine stehen aus industrieller Sicht nahezu unbegrenzt als Quelle für die Synthese von Diolen zur Verfügung, so dass metallkatalysierte Dihydroxylierungsreaktionen grundsätzlich für die Herstellung von kommerziell interessanten Produkten wie Propylenglykol, aber auch Feinchemikalien wie 1,2-Pentandiol und Pinakol eingesetzt werden könnten. Obwohl katalytische Oxidationsverfahren häufig stöchiometrischen Oxidationsprozessen ökologisch überlegen sind, werden die genannten Produkte derzeit überwiegend über nichtkatalytische Mehrstufenverfahren, z.B. stöchiometrische Reaktionen mit Persäuren oder Wasserstoffperoxid und anschließende Hydrolyse des intermediär entstehenden Epoxids hergestellt. Das beruht darauf, dass die bekannten Reoxidationsmittel für Mangan-, Ruthenium- und Osmiumoxide für eine industrielle Herstellung von Fein- und Bulkchemikalien zu teuer sind und lediglich die Darstellung extrem hochpreisiger Pharmazwischenprodukte ökonomisch erlauben.

Stöchiometrisch lassen sich die Dialkohole aus Olefinen durch Umsetzung mit KMnO₄ synthetisieren (A.J. Fatiadi, *Synthesis* **1984,** 85-127; W.P. Weber, J.P. Shepard, *Tetrahedron Lett.* **1972,** 48, 4907-4908; E. Salamci, H. Segan, Y. Sütbeyaz, M. Balci; *J. Org. Chem.* **1997,** 62, 2453-2557; B.G. Hazra, T.P. Kumar, P.L. Joshi, *Liebigs Ann. Chem.* **1997,** 1029-1034). RuO₄ ergibt Dialkohole durch stöchiometrische Umsetzung von Olefinen (L. Albarella, V. Piccialli, D. Smaldone, D. Sica, *J. Chem. Res.* **1996**, 9, 400-401) und mittels katalytischer .Reaktion mit NaJO₄ als Reoxidationsmittel (T.K.M. Shing, E.K.W. Tam, V.W.F. Tai, I.H.F. Chung, Q. Jiang, *Chem. Eur. J.* **1996**, 2, 50-57; T. Sugai, H. Okazaki, A. Kuboki, H. Ohta, *Bull. chem. Soc. Jpn*. **1997**, 70, 2535-2540; J. Angermann, K. Homann, H.-U. Reissig, R. Zimmer *Synlett* **1995**, 1014-1016; M. Desjardins, L. Brammer Jr., T. Hudlicky, *Carbohydrate Res.* **1997**, 504, 39-42; M. J. Mulvihill, M.J. Miller, *Tetrahedron* **1998**, 54, 6625-6626. Erste Arbeiten zur Dihydroxylierung mittels Osmiumoxid erfolgten stöchiometrisch (O. Makowka, *Chem. Ber.* **1908**, 45, 943; R. Criegee, *Liebigs Ann. Chem.* **1936**, 522, 75; R. Criegee, *Angew. Chem.* **1937**, 50, 153). Umsetzungen mit katalytischen Mengen Osmiumtetroxid und Chloraten als Reoxidationsmittel (K.A. Hoffmann, *Chem*. **1912**, 45, 3329) oder H₂O₂ in tert. Butanol (N.A. Milas, J.-H. Trepagnier, J.T. Nolan, M. Ji. Iliopolus, *J. Am. Chem. Soc.* **1959**, 81, 4730) als Reoxidans führen zur Überoxidation der entstehenden Diole. Bei Einsatz von H₂O₂ wird die Peroxoosmiumsäure H₂O₂O₆ gebildet, die das intermediär gebildete Diol spaltet und zu Carbonylverbindungen führt. Zur Herabsetzung der Überoxidation werden derzeit tert.-Butylhydroperoxid in Gegenwart von Et₄NOH (K.B. Sharpless, K. Akashi, *J. Am. Chem. Soc*. **1976,** 98, 1986; P.H.J. Carlsen, T. Katsuki, V.S. Martin, K.B. Sharpless, *J. Org. Chem.* **1981,** 46, 3936; F.X. Webster, J. Rivas-Enterrios, R.M. Silverstein, *J. Org. Chem*. **1987,** 52, 689; V.S. Martin, M.T. Nunez, C.E. Tonn, *Tetrahedron Lett.* **1988,** 29, 2701; M. Caron, P.R. Carlier, K.B. Sharpless, *J. Org. Chem.* **1988,** 53, 5185), tertiäre Aminoxide und in den meisten Fällen N-Methylmorpholin-N-oxid (NMO; Upjohn Prozess), W.P. Schneider, A.V. McIntosh, US 2,769,824 (1956); V. Van Rheenen, R.C. Kelly, D.Y. Cha, *Tetrahedron Lett.* **1976**, 17, 1973) als Reoxidationsmittel eingesetzt. Für trisubstituierte und teilweise auch tetrasubstituierte Alkene ist Trimethylaminoxid dem NMO überlegen (R. Ray, D.S. Matteson, *Tetrahedron Lett.* **1980,** 21, 449). Trotz der selektiven und katalytischen Dihydroxylierung, die mit N-Oxiden möglich sind, ist der Preis dieser Reoxidantien prohibitiv für größere technische Anwendungen.

In den letzten Jahren ist Na₃[Fe(CN)₆] in Gegenwart von Natriumcarbonat in einem 2-Phasensystem sehr erfolgreich als Reoxidationsmittel für OsO₄ eingesetzt worden (M. Minato, K. Yamamoto, J. Tsuji, *J. Org. Chem.* **1990**, 55, 766; M.P. Singh, H.S. Singh, B.S. Arya, A.K. Singh, A.K. Sisodia, *Indian J. Chem.* **1975**, 13, 112), insbesondere auch in der enantioselektiven Dihydroxylierung (Y. Ogino, H. Chen, H.L. Kwong, K.B. Sharpless, *Tetrahedron Lett.* **1991**, 32, 3965). Erhebliche Nachteile für eine Synthese der Diole in größerem Maßstab sind auch hier der Preis und die überstöchiometrische Menge an aufzuwendendem Eisenkomplex (3 Mol = 990 g für 1 Mol Substrat) unter Zusatz von Kaliumcarbonat (3 Mol = 420 g). Auch bei Verfahren zur elektrochemischen Oxidation des bei der Reaktion entstehenden Na₄[Fe(CN)₆] zu Na₃[Fe(CN)₆] (Sepracor Inc. (Y. Gao, C.M. Zepp), PCT Int. Appl. WO 9 317 150, 1994; Anon., *Chem. Eng. News.* **1994**, 72 (24), 41) ist eine großtechnische Umsetzung schwierig, da elektrochemische Verfahren aufgrund der benötigten apparativen Voraussetzungen generell zu teuer sind.

Um die Nachteile der genannten Reoxidantien zu umgehen, wurde in der Vergangenheit versucht, Luft bzw. Sauerstoff für die Reoxidation des Os^{VI} zu Os^{VIII} einzusetzen. Ein solches Verfahren ist von ökonomischen und ökologischen Gesichtspunkten her die attraktivste Methode. *Cairns u. Mitarb.* zeigten jedoch, dass bei der Umsetzung von Allylalkohol, Ethylen, Cyclohexen und Styren in Gegenwart von OsO₄ und Sauerstoff kein 1,2-Dialkohol gefunden wird, sonders es entstehen in allen Fällen durch Überoxidation in technisch nicht nutzbaren Mengen die entsprechenden Carbonsäuren z.B. Benzoesäure (Styren als Substrat) und CO₂ (J.F. Caims, H.L. Roberts, *J. Chem. Soc. (C)* **1968**, 640). Auch gemäß eines Verfahrens der Celanese Corporation (GB-PS 1.028.940) werden aus 1-Octen nur Ameisensäure und Heptansäure erhalten. Selbst bei der Umsetzung des nicht entsprechend oxidationsempfindlichen Ethylens erhält man nur im Spurenbereich 1,2-Diol (2 % Glykol nach 4 Stunden bei 7 MPa O₂-Druck). Mitarbeiter der *Exxon* nutzen bimetallische Systeme aus OsO₄ und Cu(II)-Salzen (US-PS 4 496 779, EP-PS 0 077 201, R.G. Austin, R.C. Michaelson, R.S. Myers in *Catalysis in Organic Ractions* (Ed: R.L. Augustine), Marcel Dekker, New York **1985**, p. 269). Analog zum Wacker-Prozess wird Os^{VI} reoxidiert durch das Cu-Salz, welches dann mittels O₂ wieder aufoxidiert wird. Maximale Umsätze von Propylen liegen hier bei 3 bis 5 % nach 2 Stunden Reaktionszeit und einem Druck von 28 bar).

Zusammenfassend lässt sich feststellen, dass die bekannten Verfahren zur Reoxidation des Osmiums, Rutheniums und Mangans mit molekularem Sauerstoff im Rahmen von Dihydroxylierungsreaktionen für Synthesen von Dialkoholen nicht nutzbar sind.

Zur Vermeidung der aufgezeigten Nachteile der bekannten katalytischen Verfahren ist es Aufgabe der Erfindung, ein neues und einfach durchzuführendes Verfahren zur metall katalysierten Dihydroxylierung zu entwickeln, das 1,2-Diole in hoher Ausbeute und Reinheit liefert, wobei molekularer Sauerstoff als Reoxidationsmittel eingesetzt wird, und das für eine großtechnische Durchführung geeignet ist.

Diese Aufgabe wird gelöst durch ein Verfahren zur Dihydroxylierung von Olefinen mittels Übergangsmetall-Katalysatoren, indem erfindungsgemäß mono-, bi- und polyfunktionelle 1,2-Diole der Formel 1,

R¹R²C(OH)-C(OH)R³R⁴ (I)

worin
- R¹ bis R⁴: unabhängig voneinander Wasserstoff, Alkyl, CN, COOH, COO-Alkyl, COO-Aryl, CO-Alkyl, CO-Aryl, O-Alkyl, O-Aryl, O-CO-Aryl, O-CO-Alkyl, OCOO-Alkyl, N-Alkyl₂, NH-Alkyl, N-Aryl₂, NH-Aryl, NO, NO₂, NOH, Aryl, Fluor, Chlor, Brom, Iod, NO₂, SiAlkyl₃, CHO, SO₃H, SO₃-Alkyl, SO₂-Alkyl, SO-Alkyl, CF₃, NHCO-Alkyl, CONH₂, CONH-Alkyl, NHCOH, NHCOO-Alkyl, CHCHCO₂-Alkyl, CHCHCO₂H, PO-(Aryl)₂, PO(Alkyl)₂, PO₃H₂, PO(O-Alkyl)₂ bedeuten und wobei Alkyl für eine aliphatische Kohlenstoffgruppe mit 1 bis 18 C-Atomen, die linear, verzweigt und/oder auch cyclisch ist, steht, und Aryl einen 4 bis zu 14 C-Atome enthaltenden fünf-, sechs- oder siebengliedrigen aromatischen Ring, wobei dieser Ring anelliert sein kann und 0 bis 3 Heteroatome wie N, O, S enthalten kann, bedeutet und wobei die Alkyl- als auch die Arylgruppe gegebenenfalls bis zu sechs weitere Substituenten tragen können, die unabhängig voneinander Wasserstoff, Alkyl, O-Alkyl, OCO-Alkyl, O-Aryl, Aryl, Fluor, Chlor, Brom, Iod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, COO-Alkyl, CONH₂, CO-Alkyl, NHCOH, NHCOO-Alkyl, CO-Aryl, COO-Aryl, PO-Aryl₂, PO₃H₂, PO(O-Alkyl)₂, SO₃-Alkyl bedeuten, wobei Alkyl und Aryl die oben genannte Bedeutung haben,
durch Umsetzung von Olefinen der allgemeinen Formel II

R¹R²C=CR³R⁴ (II)

worin
- R¹ bis R⁴: die oben genannten Bedeutungen besitzen, mit molekularem Sauerstoff in Gegenwart einer Osmium-, Ruthenium- oder Manganverbindung in Wasser oder einem Wasser enthaltenden Lösemittelgemisch bei einem pH-Wert von 7,5 bis 13 erhalten werden, wobei der Katalysator durch Zusatz eines Amins aktiviert wird.

Insbesondere werden zur Herstellung von Verbindungen der Formel I Olefine der Formel II eingesetzt, wobei die Substituenten R¹ bis R⁴ unabhängig voneinander Wasserstoff, Alkyl, CN, COOH, COO-Alkyl, COO-Aryl, CO-Alkyl, CO-Aryl, O-Alkyl, O-Aryl, N-Alkyl₂, Aryl, Fluor, Chlor, Brom lod, CHO, CF₃, NHCO-Alkyl, CONH₂, CONH-Alkyl, NHCOO-Alkyl bedeuten. Dabei hat Alkyl und Aryl die oben genannten Bedeutungen.

Besonders bevorzugt ist ein Verfahren bei dem Diole der Formel I hergestellt werden, worin R¹ bis R⁴ unabhängig voneinander Wasserstoff, Alkyl, CN, COOH, COO-Alkyl, CO-Alkyl, CO-Aryl, 0-Alkyl, O-Aryl, Aryl, Fluor, Chlor, Brom, CHO, NHCO-Alkyl, bedeuten. Dabei hat Alkyl und Aryl die oben genannten Bedeutungen.

Das erfindungsgemäße Verfahren wird in Gegenwart von Wasser durchgeführt. Als vorteilhaft hat es sich erwiesen, neben dem Olefin ein weiteres organisches Lösemittel zuzusetzen. Das erfindungsgemäße Verfahren kann bei verschiedenen Olefinen auch im Gemisch Olefin/Wasser ohne weiteres Lösemittel durchgeführt werden. Als weitere Lösungsmittel finden im Allgemeinen inerte organische Lösungsmittel Verwendung. Geeignet sind aliphatische Ether, aromatische oder aliphatische Kohlenwasserstoffe, Alkohole und Ester, halogenierte Kohlenwasserstoffe, dipolar aprotische Lösungsmittel wie Dialkylsulfoxide, N,N-Dialkylamide von aliphatischen Carbonsäuren sowie deren Gemische. Hierbei sind Alkohole, Ester und Ether bevorzugt. Als Wasserphase wird im allgemeinen eine basische wässrige Lösung mit einem pH-Wert von 7,5 bis 13 verwendet. Der basische pH-Wert der Lösung wird durch den Zusatz einer Base zum Wasser erzielt. Generell ist es vorteilhaft, in gepufferten wässrigen Lösungen, vorzugsweise bei pH 8 bis 13 zu arbeiten. Die gepufferte Lösung wird durch den Zusatz von bekannten Puffern zu Wasser zubereitet.

Mitunter ist es für die Abtrennung der Diolprodukte vorteilhaft, wenn an Stelle von Wasser oder gepufferten wässrigen Lösungen als Lösungsmittel eine wässrige Salzlösung oder gepufferte wässrige Salzlösung - beispielsweise wässrige Lösung eines Alkali- oder Erdalkalihalogenids - eingesetzt wird.

Als Oxidationsmittel wird im erfindungsgemäßen Verfahren molekularer Sauerstoff oder eine Gasmischung, die molekularen Sauerstoff enthält, eingesetzt. Bevorzugt sind Gasmischungen, die mindestens 15 Volumenprozent Sauerstoff enthalten. Besonders bevorzugt sind Luft und Sauerstoffgas mit einem Sauerstoffanteil von <95 %.

Die Reaktion läuft vorzugsweise bei Temperaturen von 20 bis 200°C ab; in vielen Fällen hat es sich bewährt, bei Temperaturen von 30 bis 150°C, bevorzugt 40 bis 100°C, zu arbeiten. Das erfindungsgemäße Verfahren kann drucklos, z.B. durch Durchleiten von Sauerstoff durch die Reaktionslösung, durchgeführt werden. Jedoch ist es für die Reaktionsgeschwindigkeit vorteilhaft, wenn ein Sauerstoffüberdruck angewandt wird. Das Verfahren kann bei Drücken bis zu 200 bar durchgeführt werden, wobei üblicherweise nur bis zu einem Druck von 60 bar und vorzugsweise im Bereich des Normaldrucks bis zu 20 bar gearbeitet wird.

Für die Selektivität der Dihydroxylierungsreaktion wird der Katalysator durch Zusatz eines Amins aktiviert. Dazu geeignet sind Amine, insbesondere tertiäre Amine wie Trialkylamine, Dialkylarylamine, Alkyldiarylamine, die cyclisch und/oder offenkettig sein können, Pyridine und Chinolone. Bevorzugt sind bicyclische Amine wie 1,4-Diazabicyclo[2,2,2]octan (DABCO) sowie Verbindungen vom Chinuclidintyp und substituierte Phthalazine, Diphenylpyrimidine und Carbamoylindoline.

Die eingesetzten Übergangsmetallkatalysatoren sind in der Regel Metalloxide der Elemente Osmium, Mangan und Ruthenium, bevorzugt Osmium. Generell werden diese Metalle in Oxidationsstufen > +4 eingesetzt. Es ist jedoch auch möglich, Präkatalysatoren in niedrigeren Oxidationsstufen einzusetzen. Diese werden unter den Reaktionsbedingungen in die katalytisch aktiven Os(VIII)- und Os(VI)-Spezies bzw. Mn(VII) oder Ru(VIII) umgewandelt. Als Osmium-Katalysatoren oder Katalysatorvorstufen können beispielsweise eingesetzt werden: OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ auf Vinylpyridin, Bu^{t}NOsO₃. Als Mangan-Katalysatoren bzw. Präkatalysatoren können beispielsweise eingesetzt werden: MnO₂, KMnO₄, Ca(MnO₄)₂, MnCl₃, Mn(OAc)₃. Als Ruthenium-Katalysatoren bzw. Präkatalysatoren können beispielsweise eingesetzt werden: RuCl₃, RuO₄, RuO₂.

Nach dem erfindungsgemäßen Verfahren wird der Katalysator in katalytischen Mengen bezüglich des Olefins eingesetzt. Generell werden zwischen 0,2 und 0,00001 Äquivalente bezogen auf Olefin, bevorzugt 0,1 bis 0,0001 und besonders bevorzugt 0,1 bis 0,0005 Äquivalente verwendet. Das Verhältnis Amin zu Metall beträgt zwischen 0,01:1 bis 1 000:1, vorzugsweise zwischen 0,1:1 bis 100:1. Besonders bevorzugt werden Verhältnisse von Amin zu Osmium von 1:1 bis 50:1 verwendet.

Bei Einsatz von sterisch anspruchsvollen Olefinen, besonders tri- und tetrasubstituierten Olefinen ist es mitunter vorteilhaft, einen Co-Katalysator zur Hydrolyse der intermediär entstehenden Metallester zuzusetzen. Dieser Co-Katalysator ist ein die Hydrolyse vereinfachendes Amid wie beispielsweise Sulfonamide oder/und Carbonsäureamide. Besonders bevorzugt ist der Zusatz von Methylsulfonsäureamid.

Der Co-Katalysator wird in einer Menge von 0,01 Mol% bis 10 Mol-% (bezogen auf Olefin) und bevorzugt von 0,1 bis 5 Mol-% eingesetzt.

Der besondere Vorteil des erfindungsgemäßen Verfahrens ist die Verwendung von Sauerstoff oder Sauerstoff enthaltenden Gasen als Reoxidationsmittel. Trotz des vergleichsweise schwierigen Reoxidationsprozesses können hohe Katalysatorproduktivitäten erreicht werden, indem die einmal eingesetzte wässrige Katalysatorphase erneut mit Olefin versetzt wird. Dadurch werden die Katalysatorkosten für das erfindungsgemäße Verfahren minimiert, so dass sogar technische Prozesse ökonomisch durchführbar sind.

Das erfindungsgemäße Verfahren ist insofern besonders überraschend und neu, da in der Vergangenheit keine katalysierten selektiven Dihydroxylierungsreaktionen zu 1,2-Diolen mit Sauerstoff als Reoxidanz beschrieben wurden. Das ist darauf zurückzuführen, dass bei den wenigen bisherigen Untersuchungen mit Sauerstoff als Reoxidanz, wenn es überhaupt zu Umsetzungen kam, im wesentlichen Überoxidationen auftraten. Die im erfindungsgemäßen Verfahren beschriebene neue Kombination von Ligandenzusatz, der die Dihydroxylierung beschleunigt, und die Durchführung des Verfahrens in einer gepufferten basischen Lösung führt überraschenderweise zu einem chemoselektiven Dihydroxylierungprozess auch in Gegenwart von Sauerstoff. Das erfindungsgemäße Verfahren zeigt hier erstmals, dass die Vorstellungen in der bekannten Literatur zur katalysierten Dihydroxylierung mit Sauerstoff falsch sind.

Die besonderen Vorteile des neuen Prozesses bestehen in dem Preisvorteil des Oxidationsmittels, der Einfachheit der Durchführung und der großen Selektivität des Verfahrens im Vergleich zu anderen verwendeten Oxidationsmitteln.

Die erfindungsgemäß hergestellten 1,2-Diole können unter anderem eingesetzt werden als Lösemittel, Ausgangsprodukte für Polyester und andere Polymere, Vorprodukte für Agrochemikalien, Kosmetika, Reinigungsmittel und finden Anwendung in Form ihrer Ester als synthetische Schmierstoffe und Weichmacher.

Die nachstehenden Beispiele dienen zur Erläuterung des erfindungsgemäßen Verfahrens, ohne es darauf zu beschränken.

### Ausführungsbeispiele

### Beispiel 1 (Vergleich)

In ein Schlenkgefäß werden 18,4 mg K₂OsO₄ x 2H₂O (0,05 mmol) eingewogen. Dazu werden unter Rühren mittels eines Magnetrührers 25 ml 0,4 bis 0,5 molarer Pufferlösung Na₃PO₄/Na₂HPO₄ vom pH 11,2 und 10 ml 2-Methyl-2-propanol gegeben, es bilden sich 2 Phasen. Das Gefäß wird in einem Wasserbad auf 50°C erwärmt und mit Sauerstoff gespült. Nach Zugabe von 173 µl Styren (1,5 mmol) wird das Reaktionsgefäß mit einer Bürette, gefüllt mit Sauerstoff, verbunden, und die Reaktionslösung wird bei 50°C unter leichtem O₂-Überdruck (ca. 50 cm Wassersäule) 24 Stunden gerührt.

Es wird wie im Folgenden beschrieben aufgearbeitet:

Zu der Reaktionslösung werden 2 g Natriumbisulfit und 10 ml Essigester gegeben. Nach 10minütigem Rühren wird die obere organische Phase abgetrennt und die wässrige Phase mit 10 ml Essigester ausgeschüttelt. Die organischen Phasen werden gereinigt, mit wasserfreiem Natriumsulfat getrocknet, im Rotationsverdampfer wird bis zur Trockne eingeengt.

Man erhält 130 mg (R)/(S)-1-Phenyl-1,2-ethandiol, 63 % (berechnet auf Styren).

Zur Isolierung eventuell entstandener saurer Produkte wird die wässrige Lösung angesäuert und 2 x mit 15 ml Ether ausgeschüttelt. Man erhält 20 mg eines kristallinen Produktes, das zu mehr als 90 % aus Benzoesäure besteht.

### Beispiel 2

Es wird wie in Beispiel 1 beschrieben, verfahren. Zu dem Osmiumsalz werden 0,05 mmol 1,4-Diazabicyclo[2,2,2]octan (DABCO) hinzugefügt. Man erhält 151 mg (R)/(S)-1-Phenyl-1,2-ethandiol (72 %) und 31 mg Benzoesäure.

### Beispiel 3 (Vergleich)

In ein Schlenkgefäß werden 7,4 mg K₂OsO₄ x 2H₂O (0,02 mmol) eingewogen. Dazu werden unter Rühren mittels eines Magnetrührers 25 ml einer Pufferlösung vom pH 10,4 dargestellt aus 0,5 molarer K₂HPO₄-Lösung und 2 molarer NaOH, sowie 10 ml 2-Methyl-2-propanol gegeben, es bilden sich 2 Phasen. Das Gefäß wird in einem Wasserbad auf 50°C erwärmt und mit Sauerstoff gespült. Nach Zugabe von 230 µl Styren (2 mmol) wird das Reaktionsgefäß mit einer Bürette, gefüllt mit Sauerstoff, verbunden, und die Reaktionslösung wird bei 50°C unter leichtem O₂-Überdruck (ca. 50 cm Wassersäule) 24 Stunden gerührt.

Es wird wie im folgenden beschrieben aufgearbeitet:

Zu der Reaktionslösung werden 2 g Natriumbisulfit und 20 ml Essigester gegeben, Nach 10 minütigem Rühren wird die obere organische Phase abgetrennt. Mittels GC werden Dialkohol sowie nicht umgesetztes Olefin bestimmt.

Ausbeute an 1-Phenyl-1,2-ethandiol: 43 % (Selektivität 57 %).

### Beispiel 4

Es wird wie in Beispiel 3 angeführt verfahren, jedoch werden zu dem Osmiumsalz 0,06 mmol 1,4-Diazabicyclo[2,2,2]octan hinzugefügt.

Ausbeute an 1-Phenyl-1,2-ethandiol: 43 % (Selektivität 78 %).

### Beispiel 5

Es wird wie im Beispiel 4 gegeben verfahren. Als Substrat werden 308 mg 2-Vinylnaphthalin (2 mmol) eingesetzt, Dialkohol sowie nicht umgesetztes Olefin werden hier mittels HPLC bestimmt.

Ausbeute an 1-(2-Naphthyl)-1,2-ethandiol: 56 % (Selektivität 75 %).

### Beispiel 6

Wie in Beispiel 3 beschrieben, jedoch unter Einsatz einer Pufferlösung vom pH 11,2 werden 7,4 mg K₂OsO₄ x 2H₂O (0,02 mmol)/ 0,02 mmol DABCO mit 318 µl 1-Phenyl-1-cyclohexen (2 mmol) umgesetzt.

Ausbeute an 1-Phenyl-1,2-cyclohexandiol: 81 % (Selektivität 84 %).

### Beispiel 7

Es wird wie in Beispiel 3 angeführt verfahren, jedoch werden zu dem Osmiumsalz 0,03 mmol 1,4-Diazabicyclo[2,2,2)octan hinzugefügt, Reaktionszeit 16 h.

Ausbeute an 2-Phenyl-1,2-propandiol: 98 % (Selektivität 98 %).

### Beispiel 8

Analog Beispiel 7 wird Cyclohexen umgesetzt, Reaktionszeit 24 h.

Ausbeute an 1,2-Cyclohexandiol: 68 % (Selektivität 75 %).

### Beispiel 9

Analog Beispiel 7 wird 1-Octen umgesetzt, Reaktionszeit 15 h.

Ausbeute an 1,2-Octandiol: 96 % (Selektivität 97 %).

### Beispiel 10

Wie in Beispiel 3 beschrieben werden 1,9 mg K₂OsO₄ x 2H₂O (0,05 mmol) unter Zusatz von 0,015 mmol 1,4-Diazabicyclo[2,2,2]octan mit 260 µl α-Methylstyren (2 mmol) umgesetzt.

Ausbeute an 2-Phenyl-1,2-propandiol: 96 % (Selektivität 96 %).

### Beispiel 11

Wie in Beispiel 3 beschrieben, jedoch in einer Reaktionszeit von 14 h und unter Einsatz einer Pufferlösung vom pH 11,2 werden 7,4 mg K₂OsO₄ x 2H₂O (0,02 mmol)/0,06 mmol DABCO mit 240 µl 1-Methyl-1-cyclohexen (2 mmol) umgesetzt.

Ausbeute an 1-Methyl-1,2-cyclohexandiol: 78 % (Selektivität 80 %).

### Beispiel 12

Analog Beispiel 11 jedoch bei pH 10,4 werden 320 µl Allyltrimethylsilan (2 mmol) umgesetzt.

Ausbeute an 3-(Trimethylsilyl)-1,2-propandiol: 72 % (Selektivität 83 %).

### Beispiel 13

Wie in Beispiel 3 beschrieben, jedoch in einer Reaktionszeit von 18 h und unter Einsatz einer Pufferlösung vom pH 12,0 werden 14,7 mg K₂OsO₄ x 2H₂O (0,04 mmol)/0,12 mmol DABCO mit 380 µl (2 mmol) *trans*-5-Decen umgesetzt.

Ausbeute an 5,6-Decandiol: 85 % (Selektivität 96 %).

### Beispiel 14

Analog Beispiel 13 werden 240 µl 2,3-Dimethyl-2-buten (2 mmol) umgesetzt.

Ausbeute an 2,3-Dimethyl-2,3-butandiol: 99 % (Selektivität 99 %).

### Beispiel 15

Analog Beispiel 13 werden 245 µl 2-Methyl-2-penten (2 mmol) bei pH 11,2 umgesetzt.

Ausbeute an 2-Methyl-2,3-pentandiol: 88 % (Selektivität 87 %).

### Beispiel 16

Analog Beispiel 13 werden 692 mg 1H,1H,2H-Perfluor-1-octen (2 mmol) bei pH 10,4 umgesetzt.

Ausbeute an 1H,1H,2H-Perfluoroctan-1,2-diol: 53 % (Selektivität 84 %).

### Beispiel 17

Analog Beispiel 16 werden 245 µl 2-Vinyl-1,3-dioxolan (2 mmol) umgesetzt.

Ausbeute an 2-(1,2-Dihydroxyethyl)-1,3-dioxolan: 59 % (Selektivität 97 %).

### Beispiel 18

Wie in Beispiel 3 beschrieben werden 7,4 mg K₂OsO₄ x 2H₂O (0,02 mmol)/0,06 mmol DABCO mit 275 µl Allylphenylether (2 mmol) umgesetzt, Reaktionszeit 18 h.

Ausbeute an 3 Phenoxy-1,2-propandiol: 50 % (Selektivität 96 %).

### Beispiel 19

In ein in einem Druckautoklaven befindliches Glasgefäß werden 0,002 mmol K₂OsO₄ x 2H₂O gelöst in Wasser 0,006 mmol DABCO und 25 ml einer Pufferlösung vom pH 10,4, dargestellt aus 0,5 molarer K₂HPO₄-Lösung und 2 molarer NaOH, sowie 12 ml 2-Methyl-2-propanol gegeben, unter Rühren mittels eines Magnetrührers bilden sich 2 Phasen. Nach Zugabe von 260 µl α-Methylstyren (2 mmol) wird ein Druck von 5 bar Sauerstoff aufgegeben und das Autoklavgefäß auf 50-55°C erwärmt.

Nach 24 h wird wie in Beispiel 3 angeführt aufgearbeitet.

Ausbeute an 2-Phenyl-1,2-propandiol: 94 % (Selektivität 94 %).

### Beispiel 20

Wie in Beispiel 19 beschrieben, werden 0,005 mmol K₂OsO₄ x 2H₂O/0,015 mmol DABCO mit 650 µl α-Methylstyren (5 mmol) bei 5 bar O₂-Druck umgesetzt.

Ausbeute an 3-Phenyl-1,2-propandiol: 95 % (Selektivität 95 %).

### Beispiel 21

Es wird wie in Beispiel 19 angeführt verfahren, jedoch werden 5 bar Pressluft anstelle von reinem Sauerstoff auf den Autoklaven gegeben.

Ausbeute an 2-Phenyl-1,2-propandiol: 41 % (Selektivität 93 %).

### Beispiel 22

Es wird wie in Beispiel 21 verfahren, jedoch werden 10 bar Pressluft auf den Autoklaven gegeben.

Ausbeute an 2-Phenyl-1,2-propandiol: 76 % (Selektivität 92 %).

## Patentansprüche

1. Verfahren zur Dihydroxylierung von Olefinen mittels Übergangsmetall-Katalysatoren zur Herstellung von mono, bi- oder/und polyfunktionellen 1,2-Diolen der Formel I,
R¹R²C(OH)-C(OH)R³R⁴ (I)
worin
R¹ bis R⁴ unabhängig voneinander Wasserstoff, Alkyl, CN, COOH, COO-Alkyl, COO-Aryl, CO-Alkyl, CO-Aryl, O-Alkyl, O-Aryl, O-CO-Aryl, O-CO-Alkyl, OCOO-Alkyl, N-Alkyl₂, NH-Alkyl, N-Aryl₂, NH-Aryl, NO, NO₂, NOH, Aryl, Fluor, Chlor, Brom, Iod, NO₂, SiAlkyl₃, CHO, SO₃H, SO₃-Alkyl, SO₂-Alkyl, SO-Alkyl, CF₃, NHCO-Alkyl, CONH₂, CONH-Alkyl, NHCOH, NHCOO-Alkyl, CHCHCO₂-Alkyl, CHCHCO₂H, PO-(Aryl)₂, PO-(Alkyl)₂, PO₃H₂, PO(O-Alkyl)₂ bedeuten und wobei Alkyl für eine aliphatische Kohlenstoffgruppe mit 1 bis 18 C-Atomen, die linear, verzweigt und/oder auch cyclisch ist, steht, und Aryl einen 4 bis zu 14 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet und wobei die Alkyl- als auch die Arylgruppe gegebenenfalls bis zu sechs weitere Substituenten tragen können, die unabhängig voneinander Wasserstoff, Alkyl, O-Alkyl, OCO-Alkyl, O-Aryl, Aryl, Fluor, Chlor, Brom, Iod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, COO-Alkyl, CONH₂, CO-Alkyl, NHCOH, NHCOO-Alkyl, CO-Aryl, COO-Aryl, PO-Aryl₂, PO₃H₂, PO(O-Alkyl)₂, SO₃-Alkyl bedeuten, wobei Alkyl und Aryl die oben genannten Bedeutungen haben, **gekennzeichnet dadurch, dass** Olefine der allgemeinen Formel II
R¹R²C=CR³R⁴ (II)
worin
R¹ bis R⁴ die oben genannten Bedeutungen besitzen, mit molekularem Sauerstoff in Gegenwart einer Osmium-, Ruthenium- oder Manganverbindung in Wasser oder einem Wasser enthaltenden Lösemittelgemisch bei einem pH-Wert von 7,5 bis 13 umgesetzt werden und der Katalysator durch Zusatz eines Amins aktiviert wird.

2. Verfahren nach Anspruch 1 zur Herstellung von Verbindungen der Formel I, **dadurch gekennzeichnet, dass** Olefine der Formel II eingesetzt werden, wobei die Substituenten R¹ bis R⁴ unabhängig voneinander Wasserstoff, Alkyl, CN, COOH, COO-Alkyl, COO-Aryl, CO-Alkyl, CO-Aryl, O-Alkyl, O-Aryl, N-Alkyl₂, Aryl, Fluor, Chlor, Brom, Iod, CHO, CF₃, NHCO-Alkyl, CONH₂, CONH-Alkyl, NHCOO-Alkyl bedeuten, und Alkyl und Aryl dabei die oben genannten Bedeutungen besitzen.

3. Verfahren nach Anspruch 1 oder 2, **dadurch gekennzeichnet, dass** Diole der Formel I hergestellt werden, worin R¹ bis R⁴ unabhängig voneinander Wasserstoff, Alkyl, CN, COOH, COO-Alkyl, CO-Alkyl, CO-Aryl, O-Alkyl, O-Aryl, Aryl, Fluor, Chlor, Brom, CHO, NHCO-Alkyl bedeuten und Alkyl und Aryl dabei die oben genannten Bedeutungen besitzen.

4. Verfahren nach einem der Ansprüche 1 bis 3, **dadurch gekennzeichnet, dass** als Oxidationsmittel Sauerstoff oder eine Gasmischung, die mindestens 15 Volumenprozent Sauerstoff enthält, verwendet wird.

5. Verfahren nach einem der Ansprüche 1 bis 4, **dadurch gekennzeichnet, dass** es in einem Wasser enthaltenden Lösemittelgemisch durchgeführt wird und als Lösemittel Alkohole, Ester oder Ether eingesetzt werden.

6. Verfahren nach einem der Ansprüche 1 bis 5, **dadurch gekennzeichnet, dass** die Reaktion bei Temperaturen von 20 bis 200°C abläuft, wobei der Druck bis zu 200 bar betragen kann.

7. Verfahren nach einem der Ansprüche 1 bis 6, **dadurch gekennzeichnet, dass** das zugesetzte Amin ein tertiäres Amin ist.

8. Verfahren nach einem der Ansprüche 1 bis 7, **dadurch gekennzeichnet, dass** Sulfonamide wie Methylensulfonsäureamid und/oder Carbonsäureamide als Co-Katalysatoren zugesetzt werden.

9. Verfahren nach einem der Ansprüche 1 bis 8, **dadurch gekennzeichnet, dass** als Katalysatoren und/oder Präkatalysatoren die Osmiumverbindungen OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ auf Vinylpyridin, Bu^{t}NOsO₃, die Manganverbindungen MnO₂, KMnO₄, Ca(MnO₄)₂, MnCl₃, Mn(OAc)₃ und die Rutheniumverbindungen RuCl₃, RuO₄, RuO₂ eingesetzt werden.

10. Verfahren nach einem der Ansprüche 1 bis 9, **dadurch gekennzeichnet, dass** die eingesetzten Katalysatoren in einer Menge zwischen 0,2 und 0,00001 Äquivalente bezogen auf Olefin verwendet werden.

11. Verfahren nach einem der Ansprüche 1 bis 10, **dadurch gekennzeichnet, dass** das Verhältnis Amin zu Metall zwischen 0,01:1 bis 1000:1 beträgt.

## Claims

1. Process for the dihydroxylation of olefins by means of transition metal catalysts to prepare monofunctional, bifunctional or/and polyfunctional 1,2-diols of the formula I,
R¹R²C(OH)-C(OH)R³R⁴ (I)
where
R¹ to R⁴ are each, independently of one another, hydrogen, alkyl, CN, COOH, COO-alkyl, COO-aryl, CO-alkyl, CO-aryl, O-alkyl, O-aryl, O-CO-aryl, O-CO-alkyl, OCOO-alkyl, N-alkyl₂, NH-alkyl, N-aryl₂, NH-aryl, NO, NO₂, NOH, aryl, fluorine, chlorine, bromine, iodine, NO₂, Si-alkyl₃, CHO, SO₃H, SO₃-alkyl, SO₂-alkyl, SO-alkyl, CF₃, NHCO-alkyl, CONH₂, CONH-alkyl, NHCOH, NHCOO-alkyl, CHCHCO₂-alkyl, CHCHCO₂H, PO-(aryl)₂, PO-(alkyl)₂, PO₃H₂, PO(O-alkyl)₂, where alkyl represents an aliphatic organic group having from 1 to 18 carbon atoms which may be linear, branched and/or cyclic and aryl is a 5-, 6- or 7-membered aromatic ring which contains from 4 to 14 carbon atoms and may be fused and contain from 0 to 3 heteroatoms such as N, O, S and where the alkyl and/or the aryl group may bear up to six further substituents selected independently from among hydrogen, alkyl, O-alkyl, OCO-alkyl, O-aryl, aryl, fluorine, chlorine, bromine, iodine, OH, NO₂, NO, Sialkyl₃, CN, COOH, CHO, SO₃H, NH₂, NH-alkyl, N-alkyl₂, PO-alkyl₂, SO₂-alkyl, SO-alkyl, CF₃, NHCO-alkyl, COO-alkyl, CONH₂, CO-alkyl, NHCOH, NHCOO-alkyl, CO-aryl, COO-aryl, PO-aryl₂, PO₃H₂, PO(O-alkyl)₂, SO₃-alkyl, where alkyl and aryl are as defined above, **characterized in that** olefins of the formula II
R¹R²C=CR³R⁴ (II)
where
R¹ to R⁴ are as defined above, are reacted with molecular oxygen in the presence of an osmium, ruthenium or manganese compound in water or a water-containing solvent mixture at a pH of from 7.5 to 13 and the catalyst is activated by addition of an amine.

2. Process according to Claim 1 for preparing compounds of the formula I, **characterized in that** olefins of the formula II in which the substitutents R¹ to R⁴ are each, independently of one another, hydrogen, alkyl, CN, COOH, COO-alkyl, COO-aryl, CO-alkyl, CO-aryl, O-alkyl, O-aryl, N-alkyl₂, aryl, fluorine, chlorine, bromine, iodine, CHO, CF₃, NHCO-alkyl, CONH₂, CONH-alkyl, NHCOO-alkyl, where alkyl and aryl are as defined above, are used.

3. Process as claimed in Claim 1 or 2, **characterized in that** diols of the formula I in which R¹ to R⁴ are each, independently of one another, hydrogen, alkyl, CN, COOH, COO-alkyl, CO-alkyl, CO-aryl, O-alkyl, O-aryl, aryl, fluorine, chlorine, bromine, CHO, NHCO-alkyl, where alkyl and aryl are as defined above, are prepared.

4. Process as claimed in any of Claims 1 to 3, **characterized in that** the oxidant used is oxygen or a gas mixture comprising at least 15 per cent by volume of oxygen.

5. Process as claimed in any of Claims 1 to 4, **characterized in that** it is carried out in a water-containing solvent mixture and alcohols, ester or ethers are used as solvents.

6. Process as claimed in any of Claims 1 to 5, **characterized in that** the reaction proceeds at temperatures of from 20 to 200°C with the pressure being able to be up to 200 bar.

7. Process as claimed in any of Claims 1 to 6, **characterized in that** the amine added is a tertiary amine.

8. Process as claimed in any of Claims 1 to 7, **characterized in that** sulphonamides such as methylenesulphonamide and/or carboxamides are added as cocatalysts.

9. Process as claimed in any of Claims 1 to 8, **characterized in that** the osmium compounds OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ on vinylpyridine, Bu^{t}NOsO₃, the manganese compounds MnO₂, KMnO₄, Ca(MnO₄)₂, MnCl₃, Mn(OAc)₃ and the ruthenium compounds RuCl₃, RuO₄, RuO₂ are used as catalysts and/or catalyst precursors.

10. Process as claimed in any of Claims 1 to 9, **characterized in that** the catalysts used are employed in amounts of from 0.2 to 0.00001 equivalents, based on olefin.

11. Process as claimed in any of Claims 1 to 10, **characterized in that** the ratio of amine to metal is from 0.01: to 1 000:1.

## Revendications

1. Procédé de dihydroxylation d'oléfines au moyen de catalyseurs de métaux de transition pour la préparation de 1,2-diols mono, bi et/ou polyfonctionnels de formule I,
R¹R²C(OH)-C(OH)R³R⁴ (I)
dans laquelle
R¹ à R⁴ indépendamment les uns des autres signifient un hydrogène, un groupe alkyle, CN, COOH, COO-alkyle, COO-aryle, CO-alkyle, CO-aryle, O-alkyle, O-aryle, O-CO-aryle, O-CO-alkyle, OCOO-alkyle, N-alkyle₂, NH-alkyle, N-aryle₂, NH-aryle, NO, NO₂, NOH, aryle, fluor, chlore, brome, iode, NO₂, Sialkyle₃, CHO, SO₃H, SO₃-alkyle, SO₂-alkyle, SO-alkyle, CF₃, NHCO-alkyle, CONH₂, CONH-alkyle, NHCOH, NHCOO-alkyle, CHCHCO₂-alkyle, CHCHCO₂H, PO-(aryle)₂, PO-(alkyle)₂, PO₃H₂, PO(O-afkyle)₂ et où alkyle est un groupe carboné aliphatique avec 1 à 18 atomes de carbone, qui est linéaire, ramifié et/ou également cyclique, et aryle est un cycle aromatique à cinq, six ou sept chaînons contenant 4 à 14 atomes de carbone, où ce cycle peut être annelé et peut contenir 0 à 3 hétéroatomes comme N, O, S, et où le groupe alkyle comme également le groupe aryle peuvent porter éventuellement jusqu'à six autres substituants, qui indépendamment les uns des autres signifient un hydrogène, un groupe alkyle, O-alkyle, OCO-alkyle, O-aryle, aryle, fluor, chlore, brome, iode, OH, NO₂, NO, Sialkyle₃, CN, COOH, CHO, SO₃H, NH₂, NH-alkyle, N-alkyle₂, PO-alkyle₂, SO₂-alkyle, SO-alkyle, CF₃, NHCO-alkyle, COO-alkyle, CONH₂, CO-alkyle, NHCOH, NHCOO-alkyle, CO-aryle, COO-aryle, PO-aryle₂, PO₃H₂, PO(O-alkyle)₂, SO₃-alkyle, où les groupes alkyle et aryle ont les significations données ci-dessus, **caractérisé en ce qu'**on fait réagir les oléfines de formule générale II
R¹R²C=CR³R⁴ (II)
dans laquelle
R¹ à R⁴ possèdent les significations données ci-dessus, avec de l'oxygène moléculaire en présence d'un composé d'osmium, de ruthénium ou de manganèse dans l'eau ou dans un solvant contenant de l'eau à un pH de 7,5 à 13 et qu'on active le catalyseur par addition d'une amine.

2. Procédé selon la revendication 1, pour la préparation de composés de formule I, **caractérisé en ce que** on utilise des oléfines de formule II, dans laquelle les substituants R¹ à R⁴ indépendamment les uns des autres signifient un hydrogène, un groupe alkyle, CN, COOH, COO-alkyle, COO-aryle, CO-alkyle, CO-aryle, O-alkyle, O-aryle, N-alkyle₂, aryle, fluor, chlore, brome, iode, CHO, CF₃, NHCO-alkyle, CONH₂, CONH-alkyle, NHCOO-alkyle, et les groupes alkyle et aryle ont les significations données ci-dessus.

3. Procédé selon la revendication 1 ou 2, **caractérisé en ce qu'**on prépare des diols de formule I, dans laquelle R¹ à R⁴ indépendamment les uns des autres signifient un hydrogène, un groupe alkyle, CN, COOH, COO-alkyle, CO-alkyle, CO-aryle, O-alkyle, O-aryle, aryle, fluor, chlore, brome, CHO, NHCO-alkyle, et les groupes alkyle et aryle ont les significations données ci-dessus.

4. Procédé selon l'une des revendications 1 à 3, **caractérisé en ce qu'**on utilise comme agent d'oxydation de l'oxygène ou un mélange gazeux qui contient au moins 15 pour-cent en volume d'oxygène.

5. Procédé selon l'une des revendications 1 à 4, **caractérisé en ce qu'**il est réalisé dans un mélange de solvant contenant de l'eau et qu'on utilise comme solvant des alcools, des esters ou des éthers.

6. Procédé selon l'une des revendications 1 à 5, **caractérisé en ce que** la réaction a lieu à des températures de 20 à 200 °C, la pression pouvant atteindre jusqu'à 200 bar.

7. Procédé selon l'une des revendications 1 à 6, **caractérisé en ce que** l'amine ajoutée est une amine tertiaire.

8. Procédé selon l'une des revendications 1 à 7, **caractérisé en ce qu'**on ajoute comme co-catalyseur des sulfonamides comme l'amide d'acide méthylènesulfonique et/ou des amides d'acide carbonique.

9. Procédé selon l'une des revendications 1 à 8, **caractérisé en ce qu'**on emploie comme catalyseurs et/ou pré-catalyseurs les composés d'osmium O_{S}O₄, K₂OS₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ sur la vinylpyridine, Bu^{t}NOsO₃, les composés de manganèse MnO₂, KMnO₄, Ca(MnO₄)₂, MnCl₃, Mn(OAc)₃ et les composés de ruthénium RuCl₃, RuO₄, RuO₂.

10. Procédé selon l'une des revendications 1 à 9, **caractérisé en ce que** les catalyseurs employés sont utilisés en une quantité entre 0,2 et 0,00001 équivalent, rapporté à l'oléfine.

11. Procédé selon l'une des revendications 1 à 10, **caractérisé en ce que** le rapport amine au métal est entre 0,001 : 1 et 1000 : 1.
